# FASCICULE DE BREVET EUROPEEN

(11) **EP 2 419 396 B1**
(45) Date de publication et mention de la délivrance du brevet: **01.05.2013**
(21) Numéro de dépôt: 10715145.8
(22) Date de dépôt: 31.03.2010
(51) Int. Cl.: C07C 51/09, C07C 59/08, C08J 11/24

(54) **RECYCLAGE CHIMIQUE DU PLA PAR ALCOOLYSE**
CHEMISCHES RECYCLINGVERFAHREN VON PLA DURCH ALKOHOLYSIERUNG
CHEMICAL RECYCLING OF PLA BY ALCOHOLYSIS

(30) Priorité: 14.04.2009 BE 200900231
(43) Date de publication de la demande: 22.02.2012
(73) Titulaire: Galactic S.A., 7760 Escanaffles (BE)
(72) Inventeur: COSZACH, Philippe, B-7760 Escanaffles (BE); BOGAERT, Jean-Christophe, B-7760 Escanaffles (BE); WILLOCQ, Jonathan, B-7760 Escanaffles (BE)
(74) Mandataire: Decamps, Alain René François
(86) Numéro de dépôt international: PCT/EP2010/054280
(87) Numéro de publication internationale: WO 2010/118955

(56) Documents cités:
- EP-A1- 0 628 533

## Description

### Domaine de l'invention

La présente invention se rapporte à un procédé de recyclage chimique ou encore dénommé dépolymérisation du polylactide (PLA) , contenu ou non dans un mélange d'autres polymères, pour en reformer son monomère ou un dérivé de celui-ci.

A l'heure actuelle, afin de promouvoir l'essor des biopolymères, dont l'utilisation s'inscrit dans une perspective de respect de l'environnement, il est essentiel de pouvoir démontrer la viabilité de la gestion de la fin de vie de ces produits. L'objectif de la présente invention est de répondre à cette problématique pour le cas du polylactide (PLA) en proposant une solution originale se démarquant de celles déjà existantes.

### Etat de l'art

La gestion de la fin de vie des matières plastiques est un élément très important dans la viabilité d'un plastique sur le marché (par exemple, le PVC a été retiré du marché des bouteilles en plastique faute d'un système de recyclage efficace). Tout comme les plastiques d'origine non renouvelable (issus de la pétrochimie) et bien que leurs filières de fm de vie soient plus nombreuses, les biopolymères sont confrontés à des défis techniques lorsqu'il s'agit de cette gestion de fin de vie. Notamment lorsque l'on parle de volumes très importants, générés dans les marchés de commodité. C'est pourquoi, il est important de traiter ce problème.

A l'heure actuelle, différentes voies permettant de gérer la fin de vie des déchets sont déjà connues tels que la mise en décharge, l'incinération, le compostage, le recyclage mécanique ou encore le recyclage chimique.

Au niveau de la mise en décharge, il a été constaté que des polluants, principalement le méthane et le dioxyde de carbone, mais aussi des pesticides, des métaux lourds et des adjuvants, sont émis lors de la dégradation en décharge. Si mettre les déchets en décharge a longtemps été une solution pratique et peu coûteuse, il a été observé, outre les émissions de polluants précitées, que les déchets continuent de se dégrader produisant des lixiviats et des gaz qui doivent continuer à être évacués et traités pendant des périodes s'étendant jusqu'à plusieurs dizaines d'années. Dans le cas des biopolymères, toutefois, la pollution est moins importante étant donné que les produits de dégradation sont moins toxiques. Il faut malgré tout prendre en considération la durée de dégradation parfois assez longue, ce qui peut être problématique lorsque les volumes à traiter sont importants.

L'objectif de l'incinération est de réduire le volume de déchets en le convertissant en gaz (CO₂, H₂O, SOₓ, HCl, NOₓ, ...), il est donc inévitable que la composition de l'air au voisinage des incinérateurs soit altérée et contienne des niveaux de substances toxiques plus élevés. Dans le cas des bioplastiques, les rejets de CO₂ sont moins problématiques car le carbone n'est pas d'origine fossile, le bilan global reste donc neutre, voire légèrement positif en tenant compte des émissions dues au procédé (biomasse vers bioplastique). Par contre les autres rejets sont plus problématiques et entrainent donc inévitablement l'altération de la composition de l'air. Bien conçus et bien exploités, les incinérateurs pourraient réduire leurs émissions mais il s'agit d'une technologie extrêmement coûteuse tant en termes d'investissements que de dépenses. L'incinération offre cependant une alternative à la mise en décharge et permet une production d'énergie, en effet, une chaudière peut récupérer la chaleur et la valoriser, éventuellement sous forme d'énergie électrique et thermique (cogénération). Les incinérateurs ayant dans le passé été sources de pollutions importantes, ils ont été nommés « centres de valorisation thermique» puis « usines de valorisation énergétique » au lieu « d'usines d'incinération ». Cependant, les dossiers d'implantation de nouvelles unités sont de plus en plus complexes à gérer car les riverains n'acceptent plus d'avoir un incinérateur à proximité de leur domicile.

La biodégradabilité, propriété importante des biopolymères, peut être valorisée avantageusement par le compostage qui ne porte pas atteinte à l'environnement lorsque les précautions nécessaires sont prises, néanmoins, la progression du matériel de départ vers le stade final dépend d'un grand nombre de facteurs externes (dimensions des matériaux, taux d'humidité, aération, pH, flore bactérienne, ratio carbone-azote) limitant parfois son utilisation. De plus, la difficulté à identifier et trier les produits contenant des polymères biodégradables (emballages alimentaires, sacs) peut détériorer la qualité du compost dans le cas où une erreur lors du tri serait effectuée. Par ailleurs, l'amélioration de la qualité du PLA (meilleure résistance thermique, meilleures propriétés mécaniques) entraîne une dégradation plus lente.

Le recyclage mécanique est également connu et utilisé, dans le cas du polyéthylène téréphtalate (PET) par exemple. Il consiste à refondre la matière, seule ou en mélange avec de la matière vierge, pour fabriquer des produits commercialisables. Les déchets sont lavés, séchés, cristallisés et broyés puis directement transformés en produits finis ou bien en granulés qui peuvent alors être commercialisés. Cette voie est également applicable au PLA. Cependant les températures utilisées étant élevées, une dégradation du polymère est souvent observée, impliquant une perte de ses propriétés mécaniques et ce, pour le PLA comme pour tout autre polymère. Le produit peut dès lors être dirigé vers des applications moins nobles ou être mélangé à de la matière vierge. Ce type de recyclage n'est donc pas infini. De plus, le recyclage pose des problèmes lorsque les plastiques sont de compositions différentes puisqu'ils ne sont généralement pas compatibles entre eux. Les températures de transformation sont en effet différentes et le mélange de plusieurs plastiques entraîne une diminution de la qualité des caractéristiques mécaniques du produit final.

Ces différentes techniques de fin de vie ne sont pas idéales car les matières plastiques ne sont pas recyclées en éléments de base (monomères) et donc directement et perpétuellement utilisables. Malgré tout, ces procédés sont viables pour le PLA mais cela uniquement si le flux de matière est composé exclusivement de PLA. En effet, si d'autres polymères contaminent le PLA, les différentes techniques précitées sont rendues difficiles. Par exemple dans le cas d'une contamination au PET, ce dernier n'est pas dégradé dans un composte. Dans le cas d'une contamination au PVC, une incinération est possible mais implique l'utilisation de filtres coûteux en raison des dégagements nocifs. Quant au recyclage mécanique, le produit obtenu est complètement dénaturé s'il est composé d'un mélange de polymères.

Une autre voie de recyclage est également connue, le recyclage chimique. Souvent citée comme la voie idéale de recyclage, il consiste à transformer le polymère par un processus chimique tel que par exemple : le craquage thermique ou catalytique en hydrocarbure, la pyrolyse qui redonne les monomères, Un système de recyclage chimique pour le PET est connu, il s'agit de sa dépolymérisation par un diol, appelé aussi glycolyse. La chaîne moléculaire est rompue et les produits obtenus sont de l'acide téréphtalique et de l'éthylène glycol. Néanmoins, certains mécanismes de dégradation lors de cette dépolymérisation, engendrent des modifications structurales irréversibles de la matière, modifications qui peuvent être responsables de difficultés lors de transformations successives. Un système de recyclage chimique du PLA peut également être envisagé afin de récupérer le monomère, l'acide lactique ou l'un de ses dérivés. Certains brevets revendiquent par exemple, une hydrolyse rapide (Brake, L.D. ; Subramanian, N.S. U.S. Patent 5,229,528, 1993) ou une solvolyse (Brake, L.D. U.S. Patent 5,264,614, 1993 ; Brake, L.D. U.S. Patent 5,264,617, 1993) de poly(hydroxy-acide) incluant le PLA avec production d'hydroxy-acides ou de leurs esters. Ces procédés connus permettent même d'arriver à un rendement proche de 95% mais ceci implique de passer par de nombreuses étapes (une estérification suivie d'une distillation, ces étapes étant répétées à trois reprises). Il s'avère cependant qu'une telle manipulation présente un risque sérieux d'une prise en masse notamment lors des étapes de distillation, ce qui rend aléatoire une transposition du procédé à l'échelle industrielle. Il s'avère également que la mise en solution dans l'alcool n'est pas des plus faciles. En effet dans le cas de l'éthanol par exemple, il n'est pas possible d'ajouter de manière continue (et donc à pression atmosphérique) le PLA à une température supérieure à 78°C (point d'ébullition de l'éthanol). De par la faible densité de certains broyats non densifiés, cela a pour conséquence une concentration limitée en PLA. Par ailleurs, le PLA alimentant le flux du recyclage chimique contient généralement de l'eau en faible quantité. Cette eau pouvant provoquer une hydrolyse de l'ester formé, libérant de cette façon de l'acide lactique. Cette production d'acide lactique est très gênante dans le cas où la qualité recherchée implique une purification par distillation avec rectification à la suite de la solvolyse. En effet, la distillation ne pourra être menée de façon optimale, la présence de l'acide lactique favorisant une oligomérisation du milieu (BE Patent BE 20080424 « Procédé continu d'obtention d'un ester lactique »). Des dégradations thermiques (pyrolyse par exemple) du PLA sont également connues menant à la formation de lactide (F.D. Kopinke, M. Remmler, K. Mackenzie, M. Möder, O. Wachsen, Polymer Degradation and stability, 53, 329-342, 1996) par un mécanisme de cyclisation par addition-élimination. Mais ces méthodes présentent un faible rendement en monomères. De plus, ces différentes techniques sont souvent réalisées à haute température et/ou haute pression ce qui provoque une dégradation chimique et optique de l'acide lactique obtenu.

Le document EP0628533 décrit un procédé de recyclage d'une source contenant un polymère tel qu'un polylactide. Ledit procédé comprend une étape de solubilisation du polymère suivie d'une dépolymérisation. Pour ces étapes le solvant utilisé peut être un alcool comprenant de 1 à 6 atomes de carbones, auquel cas lorsque la température est par la suite poussée entre 100°C et 200°C une alcoolyse a lieu pour provoque la dépolymérisation.

Il existe donc un besoin pour un procédé simple, efficace et non-dénaturant de dépolymérisation du PLA afin de pouvoir le recycler sous la forme de son monomère de base ou l'un de ses dérivés.

### Brève description de l'invention :

La présente invention a pour objet un procédé de recyclage chimique ou de dépolymérisation du PLA, contenu ou non dans un mélanges d'autres polymères, en acide lactique ou un de ses dérivés, comme un ester d'acide lactique, par alcoolyse, en conditions douces, en produisant des monomères de haute qualité et à haut rendement, en augmentant la productivité, en diminuant les émissions de CO₂ et en réduisant le coût énergétique.

Un autre objet de l'invention est de dissoudre le PLA dans un solvant du PLA qui ne bloque pas sa dépolymérisation et qui n'impose pas d'étapes supplémentaires de purification.

La présente invention a également pour objet un procédé de recyclage chimique d'un mélange de polymères contenant nécessairement du PLA, selon lequel on met le mélange en solution dans un solvant du PLA pour séparer d'abord les impuretés solides telles que les autres polymères que le PLA qui ne se sont pas dissous, et ensuite la solution de PLA est soumise à une alcoolyse afin de transformer le PLA en son monomère ou un dérivé de celui-ci.

Le procédé de la présente invention a aussi pour objet d'utiliser comme solvant pour la mise en solution du PLA, un ester lactique de manière à simplifier fortement le procédé, tout en ayant un impact positif sur toutes les étapes du procédé de recyclage chimique de l'acide polylactique.

### Description détaillée de l'invention:

La demanderesse a trouvé que la conduite d'un tel procédé de dépolymérisation pouvait être nettement améliorée si l'on effectuait au préalable une mise en solution du PLA ou du mélange de polymères contenant du PLA dans un ester lactique.

Le procédé de l'invention comprend successivement les étapes suivantes ; on effectue tout d'abord un broyage du PLA ou du mélange de polymères contenant du PLA, on utilise un ester lactique pour mettre le PLA en solution et simultanément séparer les impuretés solides telles que les autres polymères que le PLA qui ne sont pas dissous, ensuite on soumet la solution ainsi obtenue à une dépolymérisation par alcoolyse et finalement on purifie l'acide lactique ou l'un de ses dérivés obtenu de sorte à obtenir des produits répondant aux demandes spécifiques du marché traditionnel tel que les applications industrielles voir même la polymérisation du PLA.

### 1. Le broyage des déchets de PLA

Dans le cadre de l'invention présentée, les matières premières utilisées lors de ce recyclage chimique peuvent provenir de produits hors spécification dans les unités de production, de chutes de production dans les unités de transformation ainsi que de produits finis en fin de vie. On effectue d'abord le broyage du PLA ou du mélange de polymères contenant du PLA selon n'importe quelle technique connue de l'homme de l'art, comme par exemple le broyage par cisaillement, par impact, à sec ou sous eau. L'objectif de cette étape étant d'augmenter la surface spécifique des matériaux, de manière à obtenir un rapport poids/volume compris entre 0,05 et 1,4 t/m3, ce qui permet de faciliter les étapes de manutention et d'accélérer l'étape suivante de dissolution, rendant le procédé plus facilement industrialisable. Dans le cadre de l'invention, une ou plusieurs étapes de broyage peuvent être envisagées, leur nombre étant fonction du produit de départ mais également du coût de ces opérations et de la granulation finale visée. Il est également possible de pré ou post traiter ces flux de PLA ou de mélange de polymères contenant du PLA notamment en procédant à un lavage à l'eau ou autres solutions tels que par exemple solution de soude, potasse, détergente, D'autres traitements, comme un tri manuel ou encore une séparation automatique (magnétique par exemple) peuvent être envisagés, tout cela dans le but d'éliminer d'éventuels déchets qui pourraient altérer la qualité du produit final ou en compliquer la purification. Il est également évident que si les déchets de PLA ou de mélange de polymères contenant du PLA, à traiter ont une surface spécifique adéquate pour démarrer la mise en solution, on peut supprimer cette étape de broyage sans se départir du procédé de la présente invention.

Suite à cette étape de broyage, lorsqu'elle est réalisée, une étape de densification peut être envisagée afin de compacter la matière, ce qui améliorerait les étapes de manutention et de logistique.

### 2. La mise en solution du PLA ou du mélange de polymères contenant du PLA broyé

On met ensuite le mélange de polymères contenant du PLA, broyé ou non et compacté ou non, en solution avant l'étape de dépolymérisation. La mise en solution peut également être réalisée sans un broyage préalable si la forme du PLA ou du mélange de polymères contenant du PLA (rapport poids/volume) le permet. En effet, l'une des problématiques du traitement de ce type de flux est la différence de masse spécifique des différents matériaux retraités et ce même après l'étape de broyage. Bien que l'on sache qu'un avantage majeur de cette mise en solution est d'éliminer la problématique de la faible densité de matière à traiter (même lorsqu'une étape de densification est réalisée), conduisant donc à l'amélioration de la productivité par unité de volume. Il faut encore que le solvant utilisé ne soit pas gênant pour les étapes ultérieures.

Tout d'abord, ceci permet de séparer facilement les autres polymères que le PLA et de les récupérer pour un traitement spécifique, séparé et ultérieur.

De manière surprenante, on a maintenant trouvé qu'en réalisant cette mise en solution du PLA dans un ester d'acide lactique, on pouvait éviter l'étape supplémentaire ultérieure de séparation sans diminuer le rendement en matière recyclée ou dépolymérisée. Il s'agit d'esters tels que du lactate de méthyle, du lactate d'éthyle, du lactate d'isopropyle, du lactate de butyle, du lactate d'hexyle et plus généralement d'un alkyle ester d'acide lactique dont le radical alkyle a de 1 à 12 atomes de carbones. On a également trouvé que la mise en solution dans l'ester lactique avait l'avantage de pouvoir être réalisée à des températures plus importantes que celles atteintes lors de la solubilisation dans l'alcool dont cet ester est dérivé. En effet la température d'ébullition de l'ester est généralement plus haute que celle de l'alcool, ce qui permet de mettre plus de PLA en solution. De plus, cette mise en solution est assez rapide et peut-être effectuée en quelques minutes.

La Demanderesse a maintenant trouvé qu'il était possible par cette manipulation de doubler la capacité volumique en PLA et donc la quantité de matière traitée. Cette dissolution peut être préalable ou simultanée à l'étape suivante et réalisée à différentes températures allant jusqu'à la température de fusion du PLA. La société demanderesse a également mis en évidence qu'il était possible d'éliminer l'eau présente dans le PLA durant cette étape de mise en solution. En effet, compte tenu de la température d'ébullition des esters d'acide lactique préconisés dans le procédé de la présente invention la mise en solution peut être effectuée à une température de plus de 100°C et à pression atmosphérique, l'eau peut être facilement éliminée par condensation. Dans le cas d'une contamination du flux de PLA par un autre polymère (PET, PE, PVC, PP ou tout autre polymère courant), il est possible d'éliminer ce dernier par filtration si nécessaire à chaud ou tout autre moyen connu de l'homme de l'art.

En effet, les esters lactiques ne permettent pas la mise en solution des polymères précités pour les temps de traitement requis.

### 3. Le recyclage chimique du PLA

Après cette mise en solution, l'étape suivante consiste en la dépolymérisation du PLA afin de le ramener à son monomère de base (acide lactique) ou l'un de ses dérivés. Il est préférable de réaliser cette opération dans des conditions suffisamment douces afin d'éviter une dégradation de l'acide lactique ou de l'un de ses dérivés. Le fait de disposer du PLA mis en solution permet d'éviter de devoir impérativement dépasser sa température de fusion et donc de par les conditions plus douces, d'éviter ou de réduire les réactions de dégradation et de permettre ainsi d'obtenir un rendement proche de 100%.

La société demanderesse a également montré que la dépolymérisation du PLA pouvait se faire par alcoolyse à une température comprise entre 80 et 180°C, préférentiellement entre 110 et 160°C et plus préférentiellement entre 120 et 140°C, sous dépression ou une pression comprise entre la pression atmosphérique et 10 bars voire plus. Cette étape d'alcoolyse du PLA permet la production d'un ester d'acide lactique par rupture d'un lien ester du polylactide suivie de la protonation du groupe carbonyle et d'une attaque nucléophile. La protonation du groupe carbonyle est réalisée grâce à l'utilisation d'un catalyseur de transestérification, qui peut être solide ou liquide et de type acide de Lewis comme par exemple l'octoate d'étain, le lactate d'étain, l'octoate d'antimoine, l'octoate de zinc, l'APTS (acide para-toluène sulfonique), etc. ou préférentiellement basique, de la famille des Guanidines, comme par exemple le TBD (triazabicyclodécène) et ses dérivés. L'attaque nucléophile, quant à elle, est effectuée à l'aide d'un alcool. La quantité d'alcool influençant la cinétique de réaction, il est néanmoins important de maintenir un compromis permettant d'éviter l'élimination d'une quantité trop importante d'alcool lors des étapes de purification ultérieures. Dans le cadre de cette invention, peuvent être utilisés les alcools contenant 1 à 12 carbones, correspondant idéalement à l'ester utilisé pour la mise en solution, tels que le méthanol, l'éthanol, le n-butanol, l'isobutanol, le sec-butanol, le tert-butanol, le n-propanol, l'isopropanol, le 2-éthylhexanol, le 2-éthylbutanol, l'hexanol,... Il est également possible d'éliminer l'eau présente dans le PLA lors de cette étape d'alcoolyse, par traitement du reflux. L'utilisation d'un système de type Dean-Stark peut même être préconisé pour une élimination par formation d'un azéotrope hétérogène entre l'eau et l'alcool. Il peut aussi être utile de renouveler l'alcool si l'eau est éliminée par formation d'un azéotrope homogène. L'alcool contaminé pouvant être traité par toutes techniques connues de l'homme de l'art, tel que par exemple, les tamis moléculaires, la pervaporation.

L'un des modes particuliers de cette invention est la mise en solution dans l'ester lactique durant laquelle l'humidité potentiellement présente dans le PLA est éliminée par évaporation afin d'éviter l'hydrolyse des esters lactiques qui seront formés. La libération d'acide lactique et l'oligomérisation catalysée par cette même molécule sont ainsi évitées.

Un mode préféré de cette invention est la mise en solution dans l'ester lactique durant laquelle l'humidité potentiellement présente dans le PLA est éliminée par évaporation. La réaction d'alcoolyse est ensuite réalisée sur la solution exempte d'eau en présence d'un catalyseur basique afin d'éliminer tout problème lié à l'acidité lors des étapes ultérieures du procédé.

### 4. La purification de l'ester d'acide lactique formé par alcoolyse

Cette partie de l'invention consiste à la purification de l'ester d'acide lactique obtenu lors de l'alcoolyse du PLA, la pureté du produit pouvant être variable en fonction de l'utilisation visée. Il est possible d'atteindre des grades de haute qualité répondant aux critères du marché. Toute technique de purification peut être envisagée comme par exemple, les séparations solide/liquide, la distillation (rectification), la cristallisation, l'extraction, le passage sur résines ou toutes autres méthodes connues de l'homme de l'art permettant le traitement de molécules thermosensibles.

### 5. L'hydrolyse de l'ester d'acide lactique

Dans cette invention, il peut également être envisagé d'hydrolyser l'ester d'acide lactique, obtenu lors de l'alcoolyse, en acide lactique. Après l'étape de purification, l'ester d'acide lactique est récupéré afin d'être hydrolysé. Il est alors mélangé avec de l'eau en présence ou non d'un catalyseur, fixé ou non sur une résine. Préférentiellement, celui-ci sera fixé. La quantité d'eau préconisée sera minimale pour un rendement maximal, afin de diminuer la dépense énergétique lors de la concentration de l'acide lactique obtenu. Cette hydrolyse peut être réalisée à pression atmosphérique ou sous dépression, elle peut également être menée en batch ou en continu par toutes méthodes connues de l'homme de l'art telle que la distillation réactive, l'utilisation d'un réacteur à écoulement piston, ... La réaction étant :

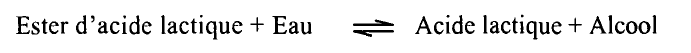

Il est nécessaire d'effectuer l'extraction de l'alcool afin de déplacer l'équilibre de la réaction vers la formation de l'acide lactique.

L'acide lactique récupéré répond aux spécifications des applications industrielles ou autres du marché. Il pourra, dans certains cas, être utilisé pour reformer du PLA.

D'autres détails et particularités de l'invention, donnés ci-après à titre d'exemples non-limitatifs, ressortent de la description comme quelques formes possibles de sa réalisation.

### Exemple 1: Recyclage de gobelets en PLA par mise en solution dans un ester lactique suivie d'une alcoolyse

1,500 kg de gobelets usagés en PLA ont été broyés à l'aide d'un broyeur à couteaux. Cette étape à permis d'augmenter la densité du volume à traiter, en effet cette dernière est passée de 0,14 à 0,25 kg/l. Ce broyat est ensuit mis en solution dans 1,000 kg de LEt à une température de 130°C sous agitation. La fin de la mise en solution est observée 5 minutes après le dernier ajout. Afin de d'éliminer l'eau potentiellement présente dans le flux de PLA, l'agitation à 130°C et à pression atmosphérique a été prolongée durant 30 minutes. Au total, c'est 11 ml d'eau qui ont été récupéré par condensation.

La solution obtenue a ensuite été transférée dans un réacteur vitrifiée permettant le travail sous pression. 1,917 kg d'éthanol sont ensuite ajoutés ainsi que 15 g de TBD.

La réaction de dépolymérisation a ensuite été menée entre 2,6 et 2,8 bars. La température maximale obtenue étant de 138°C, cette température inférieure à la température de fusion du PLA permet d'éviter une dégradation du produit. Une fois la réaction terminée, le produit a été analysé. Les résultats sont repris dans le tableau 1.

**Tableau 1: Caractéristiques de l'alcoolysat de PLA**

| H₂O^{(a)} (%) | LEt ^{(b)} (%) | EtOH ^{(b)} (%) | Acide lactique ^{(c)} (%) |
|---|---|---|---|
| 0,08 | 78,2 | 21,6 | 0,09 |

| | | | |
|---|---|---|---|
| (a) : déterminé par mesure Karl Fischer (b) : déterminé par GC lactate d'éthyle (c) : déterminé par titrage | | | |

Le produit de réaction a ensuite été purifié par distillation batch. Lors de cette distillation, deux étapes ont été observées :
- phase 1 : récupération de l'éthanol en tête de colonne
- phase 2 : récupération du lactate d'éthyle (description de cette dernière dans le tableau 2)

La quasi-totalité de la solution a été distillées. Le résidu de distillation ne représente que 2% du poids total engagé et est principalement composé des résidus de peinture, souillures et autres impuretés.

**Tableau 2: Caractéristiques de la phase lactate d'éthyle (phase 2)**

| H₂O ^{(a)} (%) | LEt ^{(b)} (%) | EtOH ^{(b)} (%) | Acide lactique (%) |
|---|---|---|---|
| 0,11 | 99,8 | N.D. | 0,07 |

| | | | |
|---|---|---|---|
| (a) : déterminé par mesure Karl Fischer (b) : déterminé par GC lactate d'éthyle (c) : déterminé par titrage | | | |

Cette manière de procédé, nous permet en une seule étape réactionnelle et une purification simple, de récupérer 98% du lactate d'éthyle attendu (solvant de mise en solution et produit de réaction), représentant un rendement de récupération du LEt provenant de la réaction de dépolymérisation d'environ 97%.

### Exemple 2 : Mise en solution dans un ester lactique

Dans le cadre de cet exemple, du PLA broyé a été mis en solution dans différents esters d'acide lactique, le lactate de méthyle, le lactate d'éthyle et le lactate de n-butyle, à l'étuve à 130°C, à pression atmosphérique et sans agitation. Les résultats de ces mises en solution sont repris dans le tableau 3.

**Tableau 3 : Mise en solution de PLA dans différents esters d'acide lactique**

| test | Ester | Rapport massique PLA/ester | temps (h) | Mise en solution complète |
|---|---|---|---|---|
| 1 | L de méthyle | 1 | 1.5 | oui |
| 2 | L d'éthyle | 1 | 2 | oui |
| 3 | L de n-butyle | 1 | 3 | oui |

La solubilisation à pression atmosphérique du PLA dans les esters lactiques ou leurs alcools respectifs ont été comparée dans l'exemple suivant.

**Tableau 4: comparaison des mises en solution du PLA dans des esters lactiques ou leurs alcools respectifs.**

| test | Solvant | Rapport massique PLA/ester | Temp. (°C) | temps (h) | Mise en solution complète |
|---|---|---|---|---|---|
| 1 | Ethanol | 1 | 78°C | 3 | non |
| 2 | L d'éthyle | 1 | 120°C | 3 | oui |
| 3 | n-butanol | 1 | 120°C | 3 | non |
| 4 | L de n-butyle | 1 | 120°C | 3 | oui |

Dans le cas du lactate d'éthyle, différents rapports ester/PLA et différentes températures ont été étudiés et comparés après une durée de 4h sans agitation à pression atmosphérique. Les résultats sont repris dans le tableau 5.

**Tableau 5: Mise en solution dans du lactate d'éthyle de PLA broyés dans différentes proportions**

| test | Rapport massique PLA/LEt | t (°C) | Mise en solution à |
|---|---|---|---|
| 1 | 0,75 | 130 | 100% |
| 2 | 1 | 130 | 100% |
| 3 | 1,5 | 130 | 100% |
| 4 | 2 | 130 | 100% |
| 5 | 1 | 120 | 100% |
| 6 | 1,25 | 120 | 100% |
| 7 | 1,5 | 120 | 100% |
| 8 | 1,75 | 120 | ~85% |
| 9 | 2 | 120 | ~75% |

Les tests 8 et 9 ont été prolongés de 2 heures. L'entièreté du PLA du test 8 est dissoute. Par contre, 10% du PLA du test 9 n'ont pas été dissous.

Une mise en solution de fibres broyées (densité = 0,22) a été réalisée dans des conditions se rapprochant des conditions industrielles (agitation, quantités de matière plus importantes, à pression atmosphérique, ...). 1,5 kg de PLA ont été mis en solution dans 1 kg de lactate d'éthyle à 130°C. La fin de la mise en solution est observée 5 minutes après le dernier ajout. La solution obtenue avait une densité d'environ 1,25.

Il a également été tenté de mettre en solution différents polymères susceptibles de pouvoir contaminer le flux de PLA, dans le lactate d'éthyle, à 130°C, à pression atmosphérique, pendant 4h et sans agitation. Les résultats sont repris dans le tableau 6.

**Tableau 6 : Mise en solution dans du lactate d'éthyle de différents polymères broyés**

| Polymère | Rapport massique polymère/LEt | Mise en solution | Aspect du mélange |
|---|---|---|---|
| PEHD | 1 | non | suspension |
| PP | 0,14 | non | suspension |
| PET | 0,37 | non | suspension |
| PLA* | 1 | oui | solution |

| | | | |
|---|---|---|---|
| * donne à titre d'exemple comparatif | | | |

Ce dernier exemple tend à prouver la possibilité de séparer les polymères contaminant le PLA par une mise en solution dans un ester d'acide lactique. Pour le confirmer, des mises en solution dans du lactate d'éthyle, de PLA contaminé par l'un de ces polymères (10%) ont été réalisées à 130°C, pendant 4h et sans agitation (rapport massique polymère/LEt = 0,5). Les insolubles sont ensuite récupérés par filtration, puis lavés abondamment à l'eau, séchés et pesés. Les résultats sont repris dans le tableau 7. Les légères différences entre les masses avant et après tentative mise en solution sont dues à la précision de la méthode utilisée.

**Tableau 7: Mise en solution dans du lactate d'éthyle de PLA contaminé par un autre polymère**

| Test | Polymère testé | Quantité de contaminant avant mise en solution | Quantité de contaminant récupéré |
|---|---|---|---|
| 1 | PEHD | 2,03 g | 2,04 g |
| 2 | PP | 1,99 g | 1,99 g |
| 3 | PET | 2,04 g | 2,03 g |

### Exemple 3 : Mise en solution dans le lactate de n-butyle, suivie d'une réaction d'alcoolyse avec le n-butanol

600 g de PLA broyé et sec ont été mis en solution dans 600 g de lactate de n-butyle. La mise en solution a été réalisée dans un ballon de 3 litres à pression ambiante et à 130°C. Afin de mimer un flux de PLA contenant un peu d'eau, 30 g d'eau ont été rajoutés. A la solution obtenue ont été ajoutés 1233 g de n-butanol et 6 g de TBD afin de réaliser la réaction d'alcoolyse (ratio molaire butanol/PLA : 2). La réaction a été menée durant 20h à pression ambiante et à 120°C (température suffisante pour solubiliser le PLA dans l'ester et douce ce qui permet d'éviter une dégradation du produit). Durant la réaction, l'eau est éliminée par condensation de l'azéotrope hétérogène formé par l'eau et le butanol. La phase butanol est réinjectée dans le ballon à l'aide d'un système de type Dean-Stark. Le résultat d'alcoolyse a ensuite été analysé et les résultats sont repris dans le tableau 8.

**Tableau 8: Caractéristiques du produit de réaction**

| Eau ^{(a)} (%) | LBut ^{(b)} (%) | Butanol ^{(b)} (%) | Acide lactique ^{(c)} (%) |
|---|---|---|---|
| 0,05 | 74,6 | 25,3 | 0,07 |

| | | | |
|---|---|---|---|
| (a) : déterminé par mesure Karl Fischer (b) : déterminés par GC lactate de butyle (c) : déterminé par titrage | | | |

Le produit de réaction a ensuite été distillé afin de récupérer le lactate de butyle formé. La distillation s'est très bien déroulée du fait de la très faible concentration en eau et en acide dans le produit de réaction. La phase lactate d'éthyle a été analysée et les résultats sont décrits dans le tableau 9.

**Tableau 9: Caractéristiques de la phase lactate de butyle**

| Eau ^{(a)} (%) | LBut ^{(b)} (%) | Butanol ^{(b)} (%) | Acide lactique ^{(c)} (%) |
|---|---|---|---|
| 0,17 | 99,7 | N.D. | 0,09 |

| | | | |
|---|---|---|---|
| (a) : déterminé par mesure Karl Fischer (b) : déterminés par GC lactate de butyle (c) : déterminé par titrage | | | |

Cette manière de procédé, nous permet en une seule étape réactionnelle et une purification simple, de récupérer plus de 97% du lactate de butyle (solvant de mise en solution et produit de réaction), représentant un rendement de récupération de l'ester lactique provenant de la réaction de dépolymérisation d'environ 96%.

### Exemple 4 : Mise en solution de PLA broyé dans du lactate d'éthyle sans élimination d'eau suivie de la réaction d'alcoolyse en présence d'éthanol

Dans un réacteur vitrifié sont placés 1,204 kg de gobelets usagé en PLA broyés préalablement mis en solution sous reflux dans 1,4 kg de lactate d'éthyle. Ensuite, 1,538 kg d'éthanol ainsi que 12 g de TBD sont rajoutés au PLA mis en solution. Ce contenu est alors chauffé durant 24h, de manière à obtenir une pression comprise entre 2,6 et 2,8 bars. La température maximale obtenue étant de 138°C, cette température inférieure à la température de fusion du PLA permet d'éviter une dégradation du produit. Une fois la réaction terminée, le produit a été analysé. Les résultats sont repris dans le tableau 10. On voit clairement que les teneurs en eau et en acide lactique en résultat sont significativement supérieures à celles observées dans l'exemple 1.

**Tableau 10 : Caractéristiques de l'alcoolysat de fibres de PLA**

| H₂O ^{(a)} (%) | LEt ^{(b)} (%) | EtOH ^{(b)} (%) | Acide lactique ^{(c)} (%) |
|---|---|---|---|
| 0,62 | 80,7 | 17,7 | 0,96 |

| | | | |
|---|---|---|---|
| (a) : déterminé par mesure Karl Fischer (b) : déterminés par GC lactate d'éthyle (c) : déterminé par titrage | | | |

Le produit de réaction a ensuite été distillé. Lors de cette distillation, trois phases ont été observées, la première étant la récupération de l'éthanol en tête de colonne. Ensuite, du lactate d'éthyle a été obtenu. Cette phase a, dans une troisième étape, été perturbée par une oligomérisation libérant des volatiles, empêchant ainsi de récupérer du lactate d'éthyle pur. La phase lactate d'éthyle a été analysée, les résultats sont repris dans le tableau 11.

**Tableau 11: Caractéristiques de la phase lactate d'éthyle (phase 2)**

| H₂O ^{(a)} (%) | LEt ^{(b)} (%) | EtOH ^{(b)} (%) | Acide lactique (%) |
|---|---|---|---|
| 0,10 | 99,8 | N.D. | 0,07 |

| | | | |
|---|---|---|---|
| (a) : déterminé par mesure Karl Fischer (b) : déterminé par GC lactate d'éthyle (c) : déterminé par titrage | | | |

Cette manière de procédé, ne nous a permis de récupérer que 61% du lactate d'éthyle théoriquement envisagé. Cet exemple montre l'importance de maîtriser l'humidité susceptible d'être présente dans le PLA.

### Exemple 5 : Hydrolyse de l'ester en acide lactique

Le lactate de butyle obtenu à l'exemple 4 a été hydrolysé afin d'en récupérer l'acide lactique. Pour ce faire, 500 g de la phase lactate de butyle obtenue ont été placés dans un ballon de 1 litre avec 123 g d'eau (ratio molaire eau/LBut : 2). La réaction a été menée à 105°C et à pression atmosphérique. Afin de pousser la réaction vers la libération de l'acide lactique, l'alcool libéré est éliminé par condensation de l'azéotrope hétérogène formé par l'eau et le butanol. L'eau est séparée du butanol à l'aide d'un Dean-Stark et réinjectée dans le ballon de réaction. La presque totalité du lactate de butyle est hydrolysé après 20 heures. Le produit obtenu répond aux critères de qualité du marché. Les résultats sont repris dans le tableau 12.

**Tableau 12: Caractéristiques de l'hydrolysat après filtration**

| H₂O ^{(a)} (%) | LBut ^{(b)} (%) | Butanol ^{(b)} (%) | Acide lactique ^{(c)} (%) |
|---|---|---|---|
| 16,7 | 0,09 | Not detected | 83,2 |

| | | | |
|---|---|---|---|
| (a) : déterminé par mesure Karl Fischer (b) : déterminé par GC lactate de butyle (c) : déterminé par titrage | | | |

### Exemple 6 : Mise en solution de PLA broyé contaminé avec du polyéthylène téréphtalate (2%) dans du lactate d'éthyle suivi de la réaction d'alcoolyse en présence d'éthanol - Elimination du contaminant après la mise en solution

1,204 kg de gobelet usagé en PLA broyés ont été contaminés par 2% de polyéthylène téréphtalate, soit 24 g. Le mélange a ensuite été mis en solution dans 1,4 kg de lactate d'éthyle à 130°C, à pression atmosphérique et sous agitation. La fin de la mise en solution a été observée 5 minutes après le dernier ajout. Afin de d'éliminer l'eau potentiellement présente dans le flux de PLA, l'agitation à 130°C et à pression atmosphérique a été prolongée durant 30 minutes. Au total, c'est 9 ml d'eau qui ont été récupérés par condensation. La solution a ensuite été filtrée à chaud afin de récupéré le PET non-dissout. Cette opération nous a permis de récupérer l'intégralité du polymère contaminant (soit 24 g).

Le filtrat a été transféré dans un réacteur vitrifié permettant le travail sous pression. 1,538 kg d'éthanol sont ensuite ajoutés ainsi que 12 g de TBD.

La réaction de dépolymérisation a ensuite été menée entre 2,6 et 2,8 bars. La température maximale obtenue étant de 136°C, cette température inférieure aux températures de fusion du PLA permet d'éviter une dégradation du produit. Une fois la réaction terminée, le produit a été analysé. Les résultats sont repris dans le tableau 13.

**Tableau 13: Caractéristiques du filtrat**

| H₂O ^{(a)} (%) | LEt ^{(b)} (%) | EtOH ^{(b)} (%) | Acide lactique ^{(c)} (%) |
|---|---|---|---|
| 0,11 | 80,9 | 18,9 | 0,07 |

| | | | |
|---|---|---|---|
| (a) : déterminé par mesure Karl Fischer (b) : déterminé par GC lactate d'éthyle (c) : déterminé par titrage | | | |

### Exemple 7 : Mise en solution de PLA broyé contaminé avec du polypropylène (1%) dans du lactate d'éthyle suivi de la réaction d'alcoolyse en présence d'éthanol - Elimination du contaminant après réaction

1,204 kg de gobelet usagé en PLA broyés ont été contaminés par 1% de polypropylène, soit 12 g. Le mélange a ensuite été mis en solution dans 1,4 kg de lactate d'éthyle à 130°C, à pression atmosphérique et sous agitation. La fin de la mise en solution a été observée 5 minutes après le dernier ajout. Afin de d'éliminer l'eau potentiellement présente dans le flux de PLA, l'agitation à 130°C et à pression atmosphérique a été prolongée durant 30 minutes. Au total, c'est 10 ml d'eau qui ont été récupérés par condensation.

La solution obtenue a ensuite été transférée dans un réacteur vitrifiée permettant le travail sous pression. 1,538 kg d'éthanol sont ensuite ajoutés ainsi que 12 g de TBD. La réaction de dépolymérisation a ensuite été menée entre 2,6 et 2,8 bars. La température maximale obtenue étant de 137°C, cette température inférieure aux températures de fusion du PLA et du PP permet d'éviter une dégradation du produit. Une fois la réaction terminée, le résultat de l'alcoolyse a été filtré afin de récupérer le polymère contaminant. Les 12 g de PP initialement introduit dans le réacteur ont été ainsi récupérés. Le filtrat a été analysé, les résultats sont repris dans le tableau 14.

**Tableau 14: Caractéristiques du filtrat**

| H₂O^{(a)} (%) | LEt ^{(b)} (%) | EtOH ^{(b)} (%) | Acide lactique ^{(c)} (%) |
|---|---|---|---|
| 0,12 | 81,2 | 18,6 | 0,09 |

| | | | |
|---|---|---|---|
| (a) : déterminé par mesure Karl Fischer (b) : déterminé par GC lactate d'éthyle (c) : déterminé par titrage | | | |

## Revendications

1. Procédé de recyclage d'un mélange de polymères contenant nécessairement du PLA, **caractérisé en ce qu'**il comprend les étapes qui consistent à :
a) Broyer et/ou compacter le mélange de polymères jusqu'à obtenir un rapport poids/volume compris entre 0,05 et 1,4 t/m3
b) Mettre en solution le mélange de polymères broyés et/ou compactés dans un solvant du PLA afin de séparer le PLA des autres polymères
c) Récupérer les polymères non dissous pour traitement séparé et ultérieur
d) Récupérer la solution de PLA avec un rapport pondéral PLA/Solvant compris entre 0,5 et 3,0 et la soumettre à une réaction catalytique d'alcoolyse, à une température comprise entre 80 et 180°C et à une pression comprise entre 0,05 et 10 bars, afin de transformer le PLA en ester lactique
e) Purifier l'ester lactique ainsi récupéré.

2. Procédé selon la revendication 1 **caractérisé en ce que** l'on effectue la mise en solution du mélange de polymères dans un ester lactique à une température comprise entre la température d'ébullition de l'eau et la température d'ébullition de l'ester à la pression de service, pendant une période de temps suffisante pour obtenir un rapport pondéral PLA/ester lactique compris entre 0,5 et 3,0 et de préférence compris entre 0,75 et 2,0.

3. Procédé selon la revendication 2 **caractérisé en ce que** la pression de service est comprise entre 0,05 et 10 bars

4. Procédé selon la revendication 2 **caractérisé en ce que** l'ester lactique est choisi parmi les lactates d'alkyle dont le radical alkyle contient de 1 à 12 atomes de carbone

5. Procédé selon la revendication 4 **caractérisé en ce que** le lactate d'alkyle est choisi parmi le lactate de méthyle, d'éthyle, d'isopropyle, de butyle ou d'hexyle.

6. Procédé selon les revendications 1 à 5 **caractérisé en ce que** l'on conduit la réaction d'alcoolyse catalytique en présence d'un catalyseur basique.

7. Procédé selon la revendication 6 **caractérisé en ce que** le catalyseur basique est choisi parmi les guanidines comme le triazabicyclodécène

8. Procédé de recyclage du PLA par dépolymérisation de celui-ci en son monomère ou un de ses dérivés, comprenant le broyage et/ou le compactage du PLA, sa mise en solution dans un solvant, l'alcoolyse catalytique du PLA dissous en ester lactique , et la purification de l'ester lactique ainsi récupéré, **caractérisé en ce que** l'on effectue la mise en solution du PLA dans un ester lactique à une température comprise entre la température d'ébullition de l'eau et la température d'ébullition de l'ester lactique à la pression de service et ce pendant une période de temps suffisante pour obtenir un rapport pondéral PLA/ester lactique compris entre 0,5 et 3,0 et de préférence compris entre 0,75 et 2,0.

9. Procédé selon la revendication 8 **caractérisé en ce que** la pression de service est comprise entre 0,05 et 10 bars

10. Procédé selon la revendication 8 **caractérisé en ce que** l'ester lactique est choisi parmi les lactates d'alkyle dont le radical alkyle contient de 1 à 12 atomes de carbone

11. Procédé selon la revendication 10 **caractérisé en ce que** le lactate d'alkyle est choisi parmi le lactate de méthyle, d'éthyle d'isopropyle, de butyle ou d'hexyle.

12. Procédé selon les revendications 8 à 11 **caractérisé en ce que** l'on conduit la réaction d'alcoolyse catalytique en présence d'un catalyseur basique.

13. Procédé selon la revendication 12 **caractérisé en ce que** le catalyseur basique est choisi parmi les guanidines comme le triazabicyclodécène

## Claims

1. Method for recycling a polymer blend necessarily containing PLA, **characterised in that** it comprises steps consisting of:
a) Grinding and/or compacting the polymer blend until a weight/volume ratio between 0.05 and 1.4 t/m3 is obtained
b) Placing the ground and/or compacted polymer blend in solution in a solvent of PLA so as to separate PLA from the other polymers
c) Retrieving the non-dissolved polymers for separate subsequent treatment
d) Retrieving the PLA solution with a PLA/Solvent weight ratio between 0.5 and 3.0 and subjecting same to a catalytic alcoholysis reaction, at a temperature between 80 and 180°C and at a pressure between 0.05 and 10 bar, so as to convert the PLA into lactic ester
e) Purifying the lactic ester retrieved.

2. Method according to claim 1, **characterised in that** the polymer blend is placed in solution in a lactic ester at a temperature between the boiling point of water and the boiling point of the ester at service pressure, for a sufficient period of time to obtain a PLA/lactic ester weight ratio between 0.5 and 3.0 and preferably between 0.75 and 2.0.

3. Method according to claim 2, **characterised in that** the service pressure is between 0.05 and 10 bar.

4. Method according to claim 2, **characterised in that** the lactic ester is chosen from alkyl lactates wherein the alkyl radical contains 1 to 12 carbon atoms.

5. Method according to claim 4, **characterised in that** the alkyl lactate is chosen from methyl, ethyl, isopropyl, butyl or hexyl lactate.

6. Method according to claims 1 to 5, **characterised in that** the catalytic alcoholysis reaction is conducted in the presence of an alkaline catalyst.

7. Method according to claim 6, **characterised in that** the alkaline catalyst is chosen from guanidines such as triazabicyclodecene.

8. Method for recycling PLA by depolymerising same into the monomer or any of the derivatives thereof, comprising grinding and/or compacting the PLA, placing same in solution in a solvent, catalytic alcoholysis of the dissolved PLA into lactic ester, and purifying the lactic ester retrieved, **characterised in that** the PLA is placed in solution in a lactic ester at a temperature between the boiling point of water and the boiling point of the ester at service pressure, for a sufficient period of time to obtain a PLA/lactic ester weight ratio between 0.5 and 3.0 and preferably between 0.75 and 2.0.

9. Method according to claim 8, **characterised in that** the service pressure is between 0.05 and 10 bar.

10. Method according to claim 8, **characterised in that** the lactic ester is chosen from alkyl lactates wherein the alkyl radical contains 1 to 12 carbon atoms.

11. Method according to claim 10, **characterised in that** the alkyl lactate is chosen from methyl, ethyl, isopropyl, butyl or hexyl lactate.

12. Method according to claims 8 to 11, **characterised in that** the catalytic alcoholysis reaction is conducted in the presence of an alkaline catalyst.

13. Method according to claim 12, **characterised in that** the alkaline catalyst is chosen from guanidines such as triazabicyclodecene.

## Patentansprüche

1. Verfahren zur Wiederverwertung einer Mischung aus Polymeren, umfassend notwendigerweise PLA, **dadurch gekennzeichnet, dass** es die Schritte umfasst, die aus Folgendem bestehen:
a) Zerkleinern und/oder Kompaktieren der Mischung aus Polymeren, bis ein Verhältnis zwischen Gewicht und Volumen erhalten wird, das zwischen 0,05 und 1,4 t/m³ liegt
b) Lösen der Mischung aus zerkleinerten und/oder kompaktierten Polymeren in einem Lösemittel der PLA, um die PLA von den anderen Polymeren zu trennen
c) Wiedergewinnen der nicht gelösten Polymere für eine getrennte und spätere Behandlung.
d) Wiedergewinnen der Lösung aus PLA mit einem Gewichtsverhältnis zwischen PLA und Lösemittel, das zwischen 0,5 und 3,0 liegt, und Unterziehen dieser einer katalytischen Alkoholyse-Reaktion bei einer Temperatur zwischen 80 und 180 °C und bei einem Druck zwischen 0,05 und 10 bar, um die PLA in Milchsäureester umzuwandeln
e) Reinigen des so wiedergewonnenen Milchsäureesters.

2. Verfahren nach Anspruch 1, **dadurch gekennzeichnet, dass** die Lösung der Mischung aus Polymeren in einem Milchsäureester bei einer Temperatur zwischen der Siedetemperatur von Wasser und der Siedetemperatur von Ester bei Betriebsdruck während eines ausreichenden Zeitraums durchgeführt wird, um ein Gewichtsverhältnis zwischen PLA und Milchsäureester zwischen 0,5 und 3,0 und vorzugsweise zwischen 0,75 und 2,0 zu erhalten.

3. Verfahren nach Anspruch 2, **dadurch gekennzeichnet, dass** der Betriebsdruck zwischen 0,05 und 10 bar liegt.

4. Verfahren nach Anspruch 2, **dadurch gekennzeichnet, dass** der Milchsäureester ausgewählt ist aus den Alkyllaktaten, deren Alkylradikal von 1 bis 12 Kohlestoffatome umfasst.

5. Verfahren nach Anspruch 4, **dadurch gekennzeichnet, dass** das Alkyllaktat ausgewählt ist aus dem Methyl-, Ethyl-, Isopropyl-, Butyl- oder Hexyllaktat.

6. Verfahren nach Anspruch 1 bis 5, **dadurch gekennzeichnet, dass** die katalytische Alkoholyse-Reaktion in Anwesenheit eines basischen Katalysators durchgeführt wird.

7. Verfahren nach Anspruch 6, **dadurch gekennzeichnet, dass** der basische Katalysator ausgewählt ist aus den Guanidinen wie z. B. dem Triazabicyclodecen.

8. Verfahren zur Wiedergewinnung der PLA durch Depolymerisation dieser in ihr Monomer oder eines ihrer Derivate, umfassend die Zerkleinerung und/oder die Kompaktierung der PLA, ihre Lösung in einem Lösemittel, die katalytische Alkoholyse der gelösten PLA in Milchsäureester und die Reinigung des so wiedergewonnenen Milchsäureesters, **dadurch gekennzeichnet, dass** die Lösung der PLA in einem Milchsäureester bei einer Temperatur zwischen der Siedetemperatur von Wasser und der Siedetemperatur von Milchsäureester beim Betriebsdruck durchgeführt wird, und dies während eines ausreichenden Zeitraums, um ein Gewichtsverhältnis zwischen PLA und Milchsäureester zwischen 0,5 und 3,0 und vorzugsweise zwischen 0,75 und 2,0 zu erhalten.

9. Verfahren nach Anspruch 8, **dadurch gekennzeichnet, dass** der Betriebsdruck zwischen 0,05 und 10 Bar liegt.

10. Verfahren nach Anspruch 8, **dadurch gekennzeichnet, dass** der Milchsäureester ausgewählt ist aus den Alkyllaktaten, deren Alkylradikal von 1 bis 12 Kohlestoffatome umfasst.

11. Verfahren nach Anspruch 10, **dadurch gekennzeichnet, dass** das Alkyllaktat ausgewählt ist aus dem Methyl-, Ethyl-, Isopropyl-, Butyl- oder Hexyllaktat.

12. Verfahren nach Anspruch 8 bis 11, **dadurch gekennzeichnet, dass** die katalytische Alkoholyse-Reaktion in Anwesenheit eines basischen Katalysators durchgeführt wird.

13. Verfahren nach Anspruch 12, **dadurch gekennzeichnet, dass** der basische Katalysator ausgewählt ist aus den Guanidinen wie z. B. dem Triazabicyclodecen.
